Europäisches Patentamt

(19) European Patent Office

Office européen des brevets

(11) EP 0 778 789 B1

# (12) FASCICULE DE BREVET EUROPEEN

(45) Date de publication et mention
de la délivrance du brevet:
**08.04.1998 Bulletin 1998/15**

(21) Numéro de dépôt: **95929916.5**

(22) Date de dépôt: **31.08.1995**

(51) Int. Cl.$^6$: **A61N 1/30**

(86) Numéro de dépôt international:
**PCT/FR95/01136**

(87) Numéro de publication internationale:
**WO 96/07449 (14.03.1996 Gazette 1996/12)**

(54) **DISPOSITIF D'IONOPHORESE POUR L'ADMINISTRATION TRANSCUTANEE D'UN PRINCIPE ACTIF A UN SUJET ASSURANT UN CONTROLE PRECIS DE LA POSOLOGIE**

IONTOPHORETISCHES GERÄT ZUR TRANSKUTANEN ABGABE VON MEDIKAMENTEN ZU EINEM PATIENTEN, DAS EINE GENAUE DOSIERUNGSKONTROLLE ERMÖGLICHT

PRECISE DOSAGE CONTROL IONOPHORESIS DEVICE FOR THE TRANSCUTANEOUS DELIVERY OF AN ACTIVE INGREDIENT TO A PATIENT

(84) Etats contractants désignés:
**AT BE CH DE DK ES FR GB GR IE IT LI LU MC NL PT SE**

(30) Priorité: **02.09.1994 FR 9410541**

(43) Date de publication de la demande:
**18.06.1997 Bulletin 1997/25**

(73) Titulaires:
- **ELF AQUITAINE**
  **92400 Courbevoie (FR)**
- **SANOFI**
  **75008 Paris (FR)**

(72) Inventeur: **MULLER, Daniel**
**F-64000 Pau (FR)**

(74) Mandataire: **Boillot, Marc et al**
**Elf Exploration Production**
**Département Propriété Industrielle**
**Tour Elf**
**EP/T/RD/DPI - Bureau 34 G 47**
**92078 Paris La Défense Cedex (FR)**

(56) Documents cités:
**EP-A- 0 060 452**     **WO-A-86/07268**
**WO-A-93/03790**     **FR-A- 2 687 321**
**US-A- 4 141 359**

## Description

L'invention concerne un dispositif d'ionophorèse pour l'administration transcutanée d'un principe actif à un sujet, lequel dispositif est agencé pour assurer un contrôle précis de la posologie.

L'ionophorèse consiste à faire passer un courant électrique entre deux électrodes équipées d'éléments réservoirs dont l'un au moins contient le principe actif à administrer, chaque élément réservoir étant placé au contact d'une zone de la peau du sujet soumis au traitement. Le principe actif, généralement sous la forme d'une solution aqueuse ou d'une dispersion dans un gel aqueux, est le plus souvent associé à l'électrode de même polarité que sa charge ionique. Le flux de principe actif à travers la peau est accru par le champ électrique résultant de l'application du courant électrique entre les électrodes et les quantités de principe actif administrées sont sensiblement proportionnelles aux quantités de courant mises en oeuvre quels que soient le type de signal électrique utilisé et l'impédance du système constitué des électrodes et de la peau.

Malgré les importantes différences d'impédance que l'on peut observer d'un sujet à l'autre, la connaissance précise des quantités de courant ayant traversé la peau à chaque instant permet d'assurer une bonne reproductibilité des doses de principe actif administrées.

L'une des techniques les plus utilisées pour connaître, voire réguler, les quantités de courant traversant les systèmes ionophorétiques consiste à faire passer entre les électrodes un courant électrique d'intensité moyenne imposée pendant une durée préréglée. Cette technique entraîne plusieurs risques, dont les deux principaux sont, d'une part, l'apparition de réactions d'oxydoréduction ou de brûlures, consécutives à des tensions électriques élevées résultant d'impédances trop fortes de la peau, et, d'autre part, l'accroissement accidentel des densités de courant pouvant devenir rédhibitoire et entraîner de graves lésions cutanées en cas de décollement partiel de l'une au moins des électrodes. Par ailleurs, la régulation de l'intensité des signaux électriques pulsés ou intermittents, qui sont fréquemment utilisés car mieux tolérés par le sujet, se prête mal à la réalisation d'ensembles électroniques assez miniaturisés pour être incorporés dans des dispositifs portatifs légers.

L'emploi de signaux électriques à tension crête ou à tension moyenne imposée est, lui, sans danger même en cas de décollement partiel des électrodes. Par contre, en raison de la grande variabilité des impédances cutanées d'un sujet à l'autre, les densités et donc les quantités totales de courant sont encore plus délicates à mesurer et donc à gérer.

La citation FR-A-2687321 décrit un dispositif d'ionophorèse agencé pour assurer la coupure du signal électrique ionophorétique après le passage d'une quantité prédéterminée de courant électrique correspondant à une quantité globale donnée correspondante de principe actif. Pour ce faire, l'une des électrodes du dispositif, dite électrode limitante, est constituée pour subir, après passage de ladite quantité prédéterminée de courant électrique, une transformation physique conduisant à un changement brusque de son potentiel sous la forme d'une surtension ou bien à l'ouverture du circuit électrique au niveau de ladite électrode. L'utilisation d'une électrode limitante pour constituer l'une des électrodes d'un dispositif d'ionophorèse représente un pas important en matière de sécurité dudit dispositif, mais elle ne permet pas de connaître l'état d'avancement du processus d'ionophorèse ni de faire appel à des signaux électriques comportant, au cours du traitement, une ou plusieurs inversions de polarité.

Afin d'éliminer les phénomènes d'électrolyse de l'eau qui sont inévitables, lorsqu'on emploie des électrodes irréversibles en un matériau tel que carbone, titane, acier inoxydable, platine, et qui entraînent des variations de pH intolérables par la peau, on a proposé de constituer au moins l'une des électrodes des dispositifs d'ionophorèse par une électrode réversible, par exemple du type argent/chlorure d'argent (EP-A-0182520).

Pour le couple argent/chlorure d'argent, les réactions aux électrodes, lors du processus d'ionophorèse, sont les suivantes :

• à la cathode : formation d'ions chlorure selon la réaction

$$AgCl + e^- \rightarrow A\overset{\circ}{g} + Cl^-$$

• à l'anode : disparition des ions chlorure selon la réaction

$$A\overset{\circ}{g} + Cl^- \rightarrow AgCl + e^-$$

Au fur et à mesure de l'avancement de la réaction, le compartiment de la cathode s'enrichit en ions chlorure tandis que le compartiment de l'anode concomitamment s'appauvrit en ces ions.

Les électrodes argent/chlorure d'argent fonctionnent comme des "capteurs" d'ions chlorure, dont le potentiel, en circuit ouvert, est donné par la loi de Nernst, et la différence de potentiel $\Delta V$, en circuit ouvert, entre les électrodes argent/chlorure d'argent d'un dispositif d'ionophorèse en cours de fonctionnement est fonction du rapport des concentrations en ions chlorure présents dans les compartiments anodique et cathodique, ledit rapport dépendant lui-même de la quantité de courant utilisée.

Bien que le passage transcutané des ions chlorure ne soit pas négligeable au cours d'un traitement ionophorétique, on a observé que les valeurs de la différence de potentiel ΔV, en circuit ouvert, entre les électrodes argent/chlorure d'argent d'un dispositif d'ionophorèse en fonctionnement étaient aisément corrélables aux quantités de courant ayant traversé le système ionophorétique et par là-même aux quantités de principe actif ayant traversé la peau, qui sont sensiblement proportionnelles à ces quantités de courant, et ce quels que soient le type du courant utilisé pour l'ionophorèse et la nature de la peau.

L'invention propose donc un dispositif d'ionophorèse, dont au moins l'une des électrodes est à base du couple électrochimique argent/chlorure d'argent et qui est agencé pour permettre la mesure, en circuit ouvert, de la différence de potentiel ΔV s'établissant entre les électrodes du dispositif en cours de traitement, ladite mesure étant réalisée au cours de courtes interruptions du signal ionophorétique, afin de connaître l'état d'avancement du traitement ionophorétique et/ou d'assurer l'arrêt ou la modification dudit traitement.

L'invention permet d'accroître la sécurité des dispositifs d'ionophorèse et contribue à assurer une meilleure connaissance des quantités de principe actif traversant la peau.

Le dispositif d'ionophorèse selon l'invention pour l'administration transcutanée d'un principe actif à un sujet, assurant un contrôle précis de la posologie, est du type comportant un premier ensemble électrode constitué d'une première électrode en contact avec un premier élément réservoir adapté, d'une part, pour contenir un électrolyte renfermant le principe actif sous une forme au moins partiellement ionisée ou sous une forme neutre et, d'autre part, pour assurer, lorsqu'il est placé au contact d'une zone de la peau du sujet, un continuum conducteur ionique entre ladite première électrode et ladite zone, un deuxième ensemble électrode constitué d'une deuxième électrode en contact avec un deuxième élément réservoir agencé pour renfermer au moins un électrolyte et pour assurer, lorsqu'il est placé au contact d'une portion de la peau du sujet, un continuum conducteur ionique entre la deuxième électrode et ladite portion, au moins l'une desdites première et deuxième électrodes étant une électrode réversible à base du couple électrochimique Ag/AgCl, et un générateur de signaux électriques ionophorétiques connectable à chacune des première et deuxième électrodes, et il se caractérise en ce qu'il comprend, en outre, un ensemble de contrôle de l'état d'avancement de l'administration transcutanée du principe actif, lequel ensemble de contrôle comporte un séquenceur programmable délivrant périodiquement des signaux de commande, un relais inverseur multivoie actionné par les signaux délivrés par le séquenceur et comprenant une première voie et une deuxième voie simultanément en position de travail ou en position de repos, la première voie connectant l'une des électrodes du dispositif d'ionophorèse soit, en position de travail, à un pôle du générateur de signaux électriques ionophorétiques ayant même signe que ladite électrode, soit en position de repos, à une sortie de mesure, à travers la deuxième voie, laquelle deuxième voie, en position de travail, connecte à la masse ladite sortie de mesure, et un circuit de mesure ou/et de contrôle connectant la sortie de mesure à l'autre électrode du dispositif et délivrant un signal représentatif d'au moins la différence de potentiel entre lesdites électrodes en circuit ouvert.

Le circuit de mesure ou/et de contrôle peut avantageusement consister en un comparateur possédant une première entrée, qui reçoit le signal représentatif de la différence de potentiel entre les électrodes en circuit ouvert, et une deuxième entrée, qui reçoit un signal représentatif d'une différence de potentiel de consigne, et fournissant, à l'égalité, un signal de sortie, lequel signal de sortie est soit (i) appliqué au séquenceur pour activer des moyens équipant ce dernier et assurant sa remise à zéro ou/et l'envoi, par ledit séquenceur au relais inverseur, d'un signal propre à faire passer le relais inverseur de la position de travail à la position de repos de manière à interrompre le circuit ionophorétique, soit (ii) appliqué à des moyens équipant le générateur de signaux électriques ionophorétiques ou associés audit générateur et assurant l'arrêt du générateur ou l'inversion de polarité ou la modification des signaux qu'il émet à la réception dudit signal de sortie du comparateur.

La différence de potentiel de consigne appliquée à la deuxième entrée du comparateur représente la différence de potentiel entre les électrodes en circuit ouvert après passage d'une quantité totale prédéterminée d'électricité, qui correspond à une quantité prédéterminée de principe actif administrée au sujet soumis à l'opération d'ionophorèse. Pour une même différence de potentiel de consigne appliquée à la deuxième entrée du comparateur, on peut modifier la quantité totale d'électricité mise en jeu et par conséquent la dose de principe actif administrée au sujet en jouant sur les concentrations initiales en ions chlorure dans les électrodes. En effet, puisque c'est la différence de concentrations en ions chlorure entre les deux électrodes qui détermine, à chaque ouverture du circuit ionophorétique, la différence de potentiel entre lesdites électrodes en circuit ouvert, on peut atteindre la même différence de potentiel de consigne pour des quantités d'électricité différentes en modifiant les teneurs initiales en chlorure dans les électrodes et particulièrement dans la positive. On peut, par exemple, choisir lesdites teneurs initiales de façon à ce que la différence de potentiel de consigne atteigne, pour une quantité totale d'électricité prédéterminée correspondant à une dose prédéterminée de principe actif administrée, la valeur zéro volt correspondant au seuil de réponse du comparateur.

Le dispositif selon l'invention peut encore comporter un amplificateur monté en série avec le générateur de signaux électriques ionophorétiques pour produire des signaux électriques ionophorétiques de tension crête plus élevée que celle des signaux fournis par le générateur. Ledit amplificateur peut constituer les moyens, qui sont associés au générateur de signaux électriques ionophorétiques et sont commandés par le signal de sortie du comparateur pour produire la modification des signaux électriques émis par ledit générateur.

L'inversion de polarité des signaux électriques ionophorétiques émis pas le générateur peut être avantageusement réalisée par un circuit inverseur de courant associé au générateur et actionné par un signal de commande, à savoir, en particulier, le signal de sortie du comparateur. Dans ce mode de réalisation utilisant un circuit inverseur de courant, le dispositif d'ionophorèse peut encore comporter un compteur d'impulsions agencé pour commander l'arrêt du traitement ionophorétique après un nombre prédéterminé d'inversions de polarité des signaux électriques ionophorétiques émis par le générateur.

Le relais inverseur de l'ensemble de contrôle peut être du type monostable, c'est-à-dire que les première et deuxième voies du relais basculent de la position de travail à la position de repos sous l'action du signal de commande délivré par le séquenceur et se maintiennent dans cette position de repos aussi longtemps que ce signal est appliqué, puis elles reviennent en position de travail lorsque ledit signal de commande cesse d'être appliqué. Le relais inverseur peut être également du type bistable, c'est-à-dire que les première et deuxième voies du relais basculent de la position de travail à la position de repos, puis reviennent de cette position de repos à la position de travail sous l'action respectivement d'un premier signal de commande, puis d'un deuxième signal de commande délivré par le séquenceur.

Le séquenceur est programmé pour actionner le relais inverseur de telle sorte que les première et deuxième voies dudit relais soient en position de travail pendant des durées $\Delta t$ prédéterminées, de préférence régulières, allant, par exemple, de 5 à 60 minutes et en position de repos pendant des durées $\delta t$ prédéterminées, de préférence régulières, allant de 0,5 à 30 minutes par exemple.

Les éléments séquenceur, relais inverseur et circuit de mesure avec ou sans comparateur peuvent se présenter sous la forme de composants microélectroniques assurant les fonctions desdits éléments et assemblés en circuit intégré pour constituer un ensemble de contrôle miniaturisé.

Comme indiqué précédemment, l'électrode d'au moins l'un des ensembles électrodes du dispositif d'ionophorèse est une électrode réversible à base du couple électrochimique Ag/AgCl. De préférence, l'électrode de chaque ensemble électrode du dispositif d'ionophorèse consiste en une électrode réversible à base dudit couple Ag/AgCl. Les électrodes réversibles à base du couple Ag/AgCl peuvent consister, par exemple, en un film d'argent chloruré utilisé tel quel ou bien déposé sur un support approprié, conducteur ou non, par exemple en un matériau tel que carbone, titane, acier inoxydable, polymère conducteur, polypropylène, PVC, polyester ou polyéthylène. On peut également faire appel à des électrodes composites formées d'une composition constituée d'un liant polymère, d'une charge conductrice pulvérulente ou fibreuse, notamment noir de carbone ou fibres courtes de graphite, et des composants argent et chlorure d'argent sous forme divisée, comme indiqué dans la citation WO-A-9116944 ou, lorsque le liant est un polymère à base d'époxy-1,2 propane et/ou d'époxy-1,2 butane, dans la demande de brevet français n° 9409231 déposée le 26.07.1994 par les Demanderesses. Lorsque l'un des ensembles électrodes du dispositif d'ionophorèse comporte une électrode, qui n'est pas à base du couple Ag/AgCl, une telle électrode peut être, par exemple, en un matériau tel que carbone, graphite, titane, platine, acier inoxydable, polymère conducteur, et tout particulièrement zinc.

L'électrolyte renfermant le principe actif, qui est présent dans l'élément réservoir du premier ensemble électrode, contient avantageusement une solution aqueuse ou un gel aqueux adhésif ou non, dans lequel le principe actif à administrer est présent sous une forme au moins partiellement ionisée ou sous une forme neutre. De même, l'électrolyte contenu dans l'élément réservoir du deuxième ensemble électrode se présente, au moins en partie, sous la forme d'une solution aqueuse ou d'un gel adhésif ou non. Ces solutions ou gels aqueux peuvent constituer la totalité de l'électrolyte présent dans l'élément réservoir considéré ou bien peuvent former une partie seulement desdits électrolytes et être alors dispersés dans un milieu non aqueux formant le reste de l'électrolyte et choisi pour ne pas interrompre le continuum conducteur ionique entre l'électrode et la peau et pour accroître la qualité de l'adhésion entre l'électrode et la peau. Ces solutions aqueuses ou gels aqueux peuvent être obtenus comme il est bien connu dans les techniques d'ionophorèse. Des exemples de gels aqueux ou de solutions aqueuses épaisses sont notamment décrits respectivement dans les citations US-A-4764164 et US-A-3163166.

Le milieu aqueux renfermant le principe actif, de même que le milieu aqueux constituant l'autre électrolyte, peuvent renfermer, si besoin est, des additifs divers et notamment des agents susceptibles de favoriser le passage transcutané du principe actif comme, par exemple, des agents vasodilatateurs et/ou des agents amphiphiles parmi lesquels on peut citer, à titre non limitatif, des composés du type alcool ou du type ester.

Si désiré, on introduit le principe actif à administrer non seulement dans l'élément réservoir du premier ensemble électrode, mais également dans l'élément réservoir du deuxième ensemble électrode et dans ce cas l'électrode de chaque ensemble électrode du dispositif d'ionophorèse consiste avantageusement en une électrode à base du couple Ag/AgCl, ce qui permet par inversion de la polarité des signaux électriques ionophorétiques appliqués aux électrodes d'administrer le principe actif alternativement à partir de l'un ou de l'autre des éléments réservoirs.

Le générateur de signaux électriques applique, entre les électrodes du dispositif d'ionophorèse, un signal électrique qui peut être soit un signal intensiométrique, c'est-à-dire un signal d'intensité moyenne imposée, par exemple intensité constante (signal intensiostatique), soit de préférence, un signal potentiométrique, c'est-à-dire un signal de tension moyenne imposée, par exemple tension constante (signal potentiostatique). Le signal électrique du type intensiostatique ou du type potentiostatique peut être continu ou pulsé avec ou sans inversion temporaire de polarité. La fréquence du signal électrique peut aller de 0 à 500 kHz et plus particulièrement de 0 à 100 kHz.

Avantageusement, la tension moyenne du signal électrique appliqué entre les deux électrodes du dispositif d'ionophorèse est choisie entre 0,1 et 50 volts et plus spécialement entre 0,3 et 20 volts de telle sorte que la densité du courant moyen généré entre lesdites électrodes ait une valeur inférieure à 5 mA/cm$^2$ et plus particulièrement inférieure ou égale à 1,5 mA/cm$^2$.

Le générateur de signaux électriques du dispositif d'ionophorèse peut être de tout type connu permettant de générer des signaux électriques d'intensité moyenne imposée ou de tension moyenne imposée, qui sont continus ou pulsés, avec ou sans inversion temporaire de polarité, et qui présentent les caractéristiques définies ci-dessus.

Le dispositif d'ionophorèse selon l'invention peut être réalisé à partir de tout dispositif d'ionophorèse connu, que l'on a modifié pour que l'électrode de l'un au moins des ensembles électrodes et, de préférence, l'électrode de chaque ensemble électrode soit une électrode à base du couple Ag/AgCl et pour y incorporer l'ensemble de contrôle selon l'invention permettant de connaître l'état d'avancement de l'administration transcutanée du principe actif.

En particulier, le dispositif selon l'invention peut être un dispositif autonome portable, à fixer par bracelet ou éventuellement à coller sur la peau, comportant au moins une électrode et de préférence deux électrodes à base du couple Ag/AgCl ayant chacune une aire inférieure à 50 cm$^2$ et plus particulièrement comprise entre 1 cm$^2$ et 40 cm$^2$, ainsi qu'un générateur miniaturisé de signaux électriques ionophorétiques et un ensemble de contrôle selon l'invention. Ainsi, un dispositif portable autonome selon l'invention peut avoir une structure analogue à celle des dispositifs d'ionophorèse portables autonomes décrits, par exemple, dans les citations US-A-4325367, US-A-4557723, EP-A-0060452 et FR-A-2509182 sous réserve qu'au moins l'une et de préférence les deux électrodes du dispositif soit à base du couple Ag/AgCl et que ledit dispositif comporte l'ensemble de contrôle selon l'invention.

Lorsque les ensembles électrodes du dispositif d'ionophorèse sont fixés à la peau au moyen d'un adhésif, ceci peut être réalisé en munissant d'une couche d'un adhésif conduisant les ions, la face, destinée à venir au contact de la peau, de l'élément réservoir de chaque ensemble électrode ou une zone entourant ladite face.

Le dispositif d'ionophorèse selon l'invention permet d'administrer à un sujet, par voie transcutanée, les divers principes actifs administrables par ionophorèse et, notamment, des molécules thérapeutiques telles que, par exemple, insuline, métoprolol, hydrocodone, tétracyclines, salbutamol, acide valproique, propanolol, arginine-vasopressine, desmopressine ou autres, tout en autorisant un contrôle de l'état d'avancement de l'administration du principe actif utilisé.

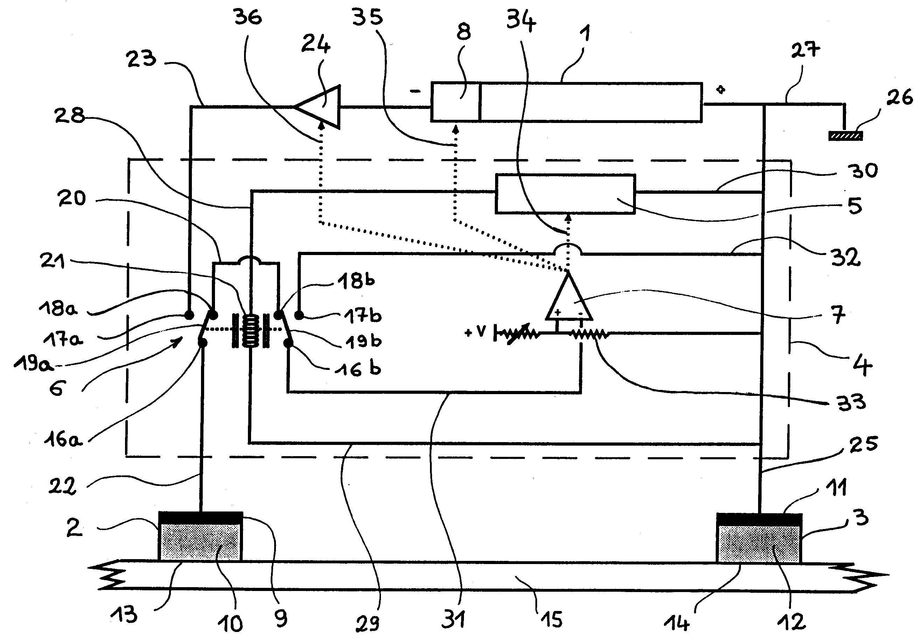

L'invention sera mieux comprise à la lecture de la description suivante d'un dispositif d'ionophorèse équipé d'un ensemble de contrôle selon l'invention. Cette description est faite en référence à la figure du dessin annexé, qui représente schématiquement un dispositif d'ionophorèse selon l'invention.

En se référant à la figure, le dispositif d'ionophorèse comporte un générateur 1 de signaux électriques ionophorétiques, un premier ensemble électrode 2, un deuxième ensemble électrode 3 et un ensemble de contrôle 4 selon l'invention, ce dernier ensemble comprenant un séquenceur 5 programmable, un relais inverseur 6 à deux voies et un circuit de mesure et/ou de contrôle constitué d'un comparateur 7.

Le générateur de signaux électriques ionophorétiques consiste en une source d'énergie électrique seule, par exemple une ou plusieurs piles primaires ou secondaires, ou en l'association d'une telle source d'énergie électrique et d'un circuit de production de signaux électriques ionophorétiques alimenté par ladite source, laquelle source d'énergie alimente également les éléments actifs du dispositif, ledit générateur incluant éventuellement un circuit inverseur de courant 8.

Le premier ensemble électrode 2 est constitué d'une électrode 9 à base du couple Ag/AgCl, par exemple un film d'argent chloruré, jouant le rôle d'électrode négative ou cathode et placée en contact avec un premier ensemble réservoir 10 renfermant un principe actif, anionique dans ce cas, en solution aqueuse. Le deuxième ensemble électrode 3 est constitué d'une électrode 11 également à base du couple Ag/AgCl, par exemple un film d'argent chloruré, jouant le rôle d'anode et disposée en contact avec un deuxième ensemble réservoir 12 renfermant un électrolyte aqueux indifférent. Le premier élément réservoir 10 et le deuxième élément réservoir 12 sont placés au contact respectivement d'une première zone 13 et d'une deuxième zone 14 de la peau 15 du sujet à traiter, lesdites zones 13 et 14 étant espacées l'une de l'autre.

Le relais inverseur 6 de l'ensemble de contrôle 4 comporte une première voie, qui est définie par un contact commun 16a, un contact de travail 17a, un contact de repos 18a et une lame de contact 19a, et une deuxième voie, qui est définie par un contact commun 16b, un contact de travail 17b, un contact de repos 18b et une lame de contact 19b, les contacts de repos 18a et 18b étant reliés entre eux par un conducteur 20. Le relais inverseur possède une position de travail, pour laquelle la lame 19a relie le contact commun 16a au contact de travail 17a et simultanément la lame 19b relie le contact commun 16b au contact de travail 17b, et une position de repos, pour laquelle la lame 19a relie le contact commun 16a au contact de repos 18a et simultanément la lame 19b relie le contact commun 16b au contact de repos 18b. Le déplacement simultané des lames 19a et 19b de la position de travail à la position de repos et vice versa est réalisé sous l'action d'une force électromagnétique créée par une bobine d'induction 21 faisant partie du relais inverseur.

Le contact commun 16a de la première voie du relais inverseur 4 est connecté à l'électrode 9 du premier ensemble électrode 2 par un conducteur 22 et le contact de travail 17a de ladite première voie est relié au pôle négatif du générateur 1 de signaux électriques ionophorétiques par un conducteur 23 à travers un amplificateur 24 à gain ajustable. L'électrode 11 du deuxième ensemble électrode 3 est connectée au pôle positif du générateur 1 par un conducteur 25 mis à la masse 26 par un conducteur 27.

La bobine 21 du relais inverseur présente une première extrémité connectée à la sortie du séquenceur 5, par un conducteur 28, et une deuxième extrémité reliée, par un conducteur 29, au conducteur 25 et de ce fait mise à la masse, le séquenceur étant également mis à la masse par un conducteur 30 reliant ledit séquenceur au conducteur 25. Le séquenceur 5 est programmé pour envoyer à la bobine d'induction 21 du relais inverseur 4, à des instants prédéterminés, des signaux d'excitation de ladite bobine pour faire passer les lames de contact 19a et 19b des première et deuxième voies du relais, de la position de travail à la position de repos et vice versa. Les excitations de la bobine 21 du relais inverseur par les signaux délivrés par le séquenceur 5 sont distribuées dans le temps de telle sorte que les première et deuxième voies du relais soient en position de travail pendant des durées $\Delta t$, de préférence égales, allant, par exemple, de 5 à 60 minutes et en position de repos pendant des durées $\delta t$, de préférence égales, allant, par exemple, de 0,5 à 30 minutes.

Le contact commun 16b de la deuxième voie du relais inverseur 4 est connecté à l'entrée négative du comparateur 7 par un conducteur 31 et le contact 17b de cette deuxième voie est relié, par un conducteur 32, au conducteur 25 et de ce fait est mis à la masse. L'entrée positive du comparateur 7 est connectée à un circuit 33 lui appliquant un signal de consigne représentant une différence de potentiel de consigne dont la valeur est ajustable. Le comparateur est agencé pour émettre, à l'égalité des signaux appliqués à ses entrées, un signal de sortie, par exemple signal 34 appliqué au séquenceur 5 pour actionner des moyens équipant ce dernier et assurant sa remise à zéro et/ou l'envoi, à la bobine 21 du relais inverseur, d'un signal propre à faire passer le relais inverseur en position de repos de manière à interrompre le circuit ionophorétique, ou bien signal 35 appliqué au circuit inverseur de courant 8 associé au générateur 1 pour inverser la polarité des signaux émis par ledit générateur ou encore signal 36 appliqué à l'amplificateur 24 à gain ajustable pour modifier le gain dudit amplificateur et donc l'amplitude des signaux qui le traversent. Le signal de sortie du comparateur peut être encore appliqué à des moyens, non représentés, équipant le générateur 1 et assurant l'arrêt dudit générateur à la réception dudit signal de sortie.

Le dispositif qui vient d'être décrit fonctionne comme indiqué ci-après.

En période de travail les deux voies du relais inverseur 4 sont en position de travail et le comparateur 7 constituant le circuit de mesure et/ou de contrôle n'est pas connecté, l'entrée négative dudit comparateur étant à la masse. Les signaux électriques ionophorétiques émis par le générateur 1 arrivent à l'une des électrodes et circulent vers l'autre électrode à travers la peau et les tissus du sujet soumis au traitement, pour revenir ensuite au générateur 1. Dans le circuit ionophorétique ainsi créé, les ions du principe actif contenu dans l'élément réservoir 10 du premier ensemble électrode 2 migrent à travers la peau et les tissus du sujet vers l'ensemble électrode 3 et passent ainsi dans le système circulatoire dudit sujet. Après un certain temps $\Delta t$ de fonctionnement en période de travail pendant laquelle une certaine quantité de principe actif migre à travers la peau du sujet, le séquenceur 5 active la bobine d'induction 21 du relais inverseur 4 pour faire passer les deux voies du relais en position de repos. Ceci entraîne la coupure du circuit ionophorétique et la connexion de l'électrode 9 de l'ensemble électrode 2 à l'entrée négative du comparateur 7, ce qui correspond à l'application, à ladite entrée négative, d'un signal représentant la différence de potentiel, en circuit ouvert, entre les électrodes 9 et 11 du dispositif d'ionophorèse. Ledit signal est comparé, dans le comparateur 7, au signal de consigne représentant une différence de potentiel prédéterminée correspondant à la quantité totale d'électricité nécessaire pour administrer une quantité totale donnée de principe actif au sujet soumis au traitement d'ionophorèse.

A l'issue de chaque période de repos $\delta t$, si le comparateur n'a pas émis de signal de sortie résultant de l'égalité entre les signaux appliqués à ses entrées, les deux voies du relais inverseur sont ramenées en position de travail sous l'action du séquenceur agissant sur la bobine d'induction 21, ce qui rétablit le circuit ionophorétique et la poursuite de l'administration ionophorétique du principe actif au sujet.

Lorsque le comparateur 7, après une série de cycles comportant chacun une période de travail $\Delta t$ suivie d'une période de repos $\delta t$, détecte l'égalité du signal représentatif de la différence de potentiel en circuit ouvert entre les électrodes et du signal représentatif de la différence de potentiel de consigne, il émet un signal de sortie utilisé comme indiqué précédemment, par exemple un signal 34 appliqué au séquenceur 5 pour actionner des moyens équipant ce dernier et assurant sa remise à zéro et, par action sur la bobine 21 du relais inverseur 4, l'interruption du circuit ionophorétique par passage des deux voies du relais en position de repos.

Pour compléter la description qui précède, on donne ci-après, à titre non limitatif, un exemple de mise en oeuvre d'un dispositif d'ionophorèse selon l'invention opérant à tension constante.

6

**EXEMPLE :**

On étudiait l'état d'avancement de la diffusion transcutanée de valproate de sodium par ionophorèse, en utilisant un dispositif d'ionophorèse pourvu de l'ensemble de contrôle selon l'invention.

On opérait dans des cellules d'ionophorèse de structure identique. Chaque cellule d'ionophorèse était constituée de trois compartiments cylindriques adjacents coaxiaux de 2 cm$^2$ de section transversale, à savoir, dans cet ordre, un compartiment donneur (compartiment cathodique), un compartiment receveur et un compartiment de contre-électrode (compartiment anodique), ces trois compartiments étant séparés, chacun du suivant et de façon étanche, par un morceau de peau de rat nu (OFA hr/hr) servant de membrane pour l'étude de la diffusion transcutanée.

Le compartiment donneur, d'un volume de 0,5 ml, renfermait une solution aqueuse à 5% en poids de valproate de sodium additionnée de 500 ppm en poids de NaN3. Le compartiment receveur, d'un volume de 10 ml, renfermait du sérum physiologique additionné de 500 ppm en poids de NaN3 et était agité à l'aide d'un barreau magnétique. Le compartiment de contre-électrode, identique au compartiment donneur, renfermait 0,5 ml d'une solution aqueuse à 2% en poids de NaCl ainsi que 500 ppm en poids de NaN3. A leur extrémité opposée au compartiment receveur, le compartiment donneur et le compartiment de contre-électrode étaient équipés, chacun, d'une électrode présentant une face active, tournée du côté de la membrane en peau de rat correspondante et ayant une surface active de 2 cm$^2$, et une face masquée par un film plastique adhésif isolant, ladite électrode consistant en un film d'argent chloruré électrochimiquement présentant une épaisseur de 15 µm et renfermant une quantité de chlorure d'argent correspondant à une quantité d'électricité égale à 1,25 mAh/cm$^2$. La surface effective d'échange était de 2 cm$^2$ pour chaque peau.

Les échantillons de peau de rat avaient été débarrassés des tissus sous-cutanés et conservés par congélation à -40°C jusqu'à leur montage dans la cellule d'ionophorèse, faces dermiques tournées vers le compartiment receveur, après un passage de 15 minutes à température ambiante dans du sérum physiologique additionné de 0,05% en poids de NaN3.

Le dispositif d'ionophorèse associé à la cellule était similaire à celui représenté sur la figure avec le compartiment donneur (compartiment cathodique) et l'électrode d'argent chloruré associée constituant le premier ensemble électrode 2 (ensemble cathodique), dont l'électrode 9 (cathode) est connectée au contact commun 16a de la première voie du relais inverseur 4, et le compartiment de contre-électrode et l'électrode d'argent chloruré associée formant le deuxième ensemble électrode 3 (ensemble anodique). Le générateur 1 de signaux électriques ionophorétiques, connecté par son pôle négatif au contact de travail 17a de la première voie du relais inverseur 4 et par son pôle positif à l'électrode 11 (anode) du deuxième ensemble électrode 3, délivrait des signaux pulsés carrés ayant une tension crête égale à 6 volts (tension moyenne constante égale à 3 volts) et une fréquence égale à 10 kHz. Le séquenceur 5 était programmé pour réaliser des cycles successifs comportant chacun une période de travail Δt égale à 15 minutes suivie d'une période de repos δt égale à 5 minutes. Le circuit de mesure ou/et de contrôle comportait le comparateur 7, commandant le séquenceur 5 pour assurer la coupure du circuit ionophorétique dès que la différence de potentiel en circuit ouvert ΔV entre les électrodes était positive ou nulle à l'issue d'une période de repos δt, et en outre un millivoltmètre monté dans ledit circuit pour permettre la mesure de la valeur ΔV pendant les périodes successives de repos.

Pour cette étude, on utilisait quatre cellules d'ionophorèse (cellules A à D) équipées et fonctionnant comme indiqué ci-dessus, lesdites cellules étant mises en marche simultanément.

Pour chaque cellule, on notait la valeur des grandeurs suivantes :

- durée séparant la fin de la période de repos de chaque cycle du début de l'opération ;
- intensité moyenne Im du courant ayant traversé la cellule au cours de la période de travail de chaque cycle ;
- différence de potentiel ΔV en fin de période de repos de chaque cycle.

On déterminait également la quantité totale d'électricité Q ayant traversé le circuit ionophorétique au cours de l'expérience, la quantité P de principe actif (valproate) contenue dans le compartiment receveur 20 heures après le début de l'expérience, ainsi que l'impédance moyenne Rm de la cellule sur la durée totale d'application du courant ionophorétique.

Les résultats obtenus sont rassemblés dans le tableau ci-après :

TABLEAU

| N° du cycle | D | Cellule A | | Cellule B | | Cellule C | | Cellule D | |
|---|---|---|---|---|---|---|---|---|---|
| | | Im ($\mu$A) | $\Delta$V (mv) | Im ($\mu$A) | $\Delta$V (mv) | Im ($\mu$A) | $\Delta$V (mv) | Im ($\mu$A) | $\Delta$V (mV) |
| | 0 | | -40 | | -40 | | -50 | | -35 |
| 1 | 0h20 | 480 | -25 | 745 | -24 | 380 | -35 | 420 | -25 |
| 2 | 0h40 | 500 | -13 | 780 | -11 | 395 | -25 | 440 | -17 |
| 3 | 1h | 510 | -7,2 | 815 | 0 * | 415 | -17 | 465 | -12 |
| 4 | 1h20 | 525 | -3,4 | | | 440 | -11 | 475 | -7,6 |
| 5 | 1h40 | 535 | +0,5 * | | | 465 | -7,4 | 490 | -3,8 |
| 6 | 2h | | | | | 495 | -3,3 | 505 | +1 * |
| 7 | 2h20 | | | | | 470 | +0,5 | * | |
| Q (mAh) | | 0,85 | | 0,78 | | 1,03 | | 0,93 | |
| P ($\mu$g) | | 275 | | 267 | | 288 | | 292 | |
| Rm ($\Omega$) | | 5800 | | 3800 | | 7820 | | 6450 | |

*Arrêt du signal*

L'examen des résultats présentés dans le tableau ci-dessus amène les remarques suivantes :

- pour une même tension appliquée, les densités de courant croissent au cours du temps pour chacune des cellules traduisant une diminution de l'impédance de ces dernières ; les impédances moyennes Rm calculées sur la durée totale d'application du signal ionophorétique sont très différentes d'une cellule à l'autre (3,8 à 7,8 k$\Omega$) ;
- malgré ces différences importantes d'impédance, qui conduisent à des densités de courant et à des durées d'activité du signal ionophorétique très variables, on constate que les quantités totales d'électricité Q ayant traversé chaque cellule sont très voisines, ce qui montre que le système a bien fonctionné comme un compteur électrochimique ;
- les quantités de principe actif ayant diffusé en 20 heures sont très proches les unes des autres, ce qui montre que les rendements électrochimiques sont quasi constants.

On a pu ainsi obtenir avec un dispositif à tension moyenne constante, très simple sur le plan de l'électronique et très sûr et agréable du point de vue de la tolérance cutanée, une bonne reproductibilité des doses de principe actif administrées.

**Revendications**

1. Dispositif d'ionophorèse pour l'administration transcutanée d'un principe actif à un sujet, assurant un contrôle précis de la posologie, du type comportant un premier ensemble électrode (2) constitué d'une première électrode (9) en contact avec un premier élément réservoir (10) adapté, d'une part, pour contenir un électrolyte renfermant le principe actif sous une forme au moins partiellement ionisée ou sous une forme neutre et, d'autre part, pour assurer, lorsqu'il est placé au contact d'une zone (13) de la peau (15) du sujet, un continuum conducteur ionique entre ladite première électrode et ladite zone, un deuxième ensemble électrode (3) constitué d'une deuxième électrode (11) en contact avec un deuxième élément réservoir (12) agencé pour renfermer au moins un électrolyte et pour assurer, lorsqu'il est placé au contact d'une portion (14) de la peau (15) du sujet, un continuum conducteur ionique entre la deuxième électrode et ladite portion, au moins l'une desdites première et deuxième électrodes étant une électrode reversible à base du couple électrochimique Ag/AgCl, et un générateur (1) de signaux électriques ionophorétiques connectable à chacune des première et deuxième électrodes, caractérisé en qu'il comprend, en outre, un ensemble (4) de contrôle de l'état d'avancement de l'administration transcutanée du principe actif, lequel ensemble de contrôle comporte un séquenceur (5) programmable délivrant périodiquement des signaux de commande, un relais inverseur (6) multivoie actionné par les signaux délivrés par le séquenceur et comprenant une première voie et une deuxième voie simultanément en position de travail ou en position de repos, la première voie connectant l'une (9) des électrodes du dispositif d'ionophorèse soit, en position de travail (16a,17a,19a), à un pôle du générateur (1) de signaux électriques ionophorétiques ayant même signe que ladite électrode, soit, en position de repos (16a,18a,19a), à une sortie de mesure (16b), à travers la deuxième voie (18b,19b,16b), laquelle deuxième voie, en position de travail (16b,17b,19b) connecte à la masse (26) ladite sortie de mesure, et un circuit de mesure ou/et de contrôle (7,33) connectant la sortie de mesure (16b) à l'autre électrode (11) du dispositif et délivrant un signal (31) représentatif d'au moins la différence de potentiel entre lesdites électrodes (9,11) en circuit ouvert.

2. Dispositif selon la revendication 1, caractérisé en ce que le circuit de mesure ou/et de contrôle consiste en un comparateur (7) possédant une première entrée, qui reçoit le signal (31) représentatif de la différence de potentiel entre les électrodes (9,11) en circuit ouvert, et une deuxième entrée, qui reçoit un signal (33) représentatif d'une différence de potentiel de consigne, et fournissant, à l'égalité, un signal de sortie (34,35 ou 36), lequel signal de sortie est soit(i) appliqué (34) au séquenceur (5) pour activer des moyens équipant ce dernier et assurant sa remise à zéro ou/et l'envoi, par ledit séquenceur au relais inverseur (6), d'un signal (28) propre à faire passer le relais inverseur de la position de travail à la position de repos de manière à interrompre le circuit ionophorétique, soit (ii) appliqué (35 ou 36) à des moyens équipant le générateur (1) de signaux électriques ionophorétiques ou associés audit générateur et agencés pour assurer l'arrêt du générateur ou l'inversion de polarité ou la modification des signaux qu'il émet, à la réception du signal de sortie du comparateur.

3. Dispositif selon la revendication 1 ou 2, caractérisé en ce qu'il comporte, en outre, un amplificateur (24) monté en série avec le générateur de signaux électriques ionophorétiques pour produire des signaux électriques ionophorétiques de tension crête plus élevée que celle des signaux fournis par le générateur.

4. Dispositif selon la revendication 2, caractérisé en ce que les moyens associés au générateur et actionnés par le signal de sortie du comparateur (7) pour modifier les signaux émis par le générateur consistent en un amplificateur (24) monté en série avec ledit générateur et commandé par le signal de sortie (36) dudit comparateur.

5. Dispositif selon l'une des revendications 1 à 4, caractérisé en ce qu'il comporte un circuit inverseur de courant (8) associé au générateur (1) et assurant l'inversion de polarité des signaux électriques ionophorétiques émis par ledit générateur.

6. Dispositif selon l'une des revendications 2 à 4, caractérisé en ce que les moyens associés au générateur et auxquels on applique le signal de sortie du comparateur (7) pour assurer l'inversion de polarité des signaux électriques ionophorétiques émis par ledit générateur consistent en un circuit inverseur de courant (8) activé par ledit signal de sortie (35).

7. Dispositif selon la revendication 5 ou 6, caractérisé en ce qu'il comprend, également, un compteur d'impulsions agencé pour commander l'arrêt du traitement ionophorétique après un nombre prédéterminé d'inversions de polarité des signaux électriques ionophorétiques émis par le générateur.

8. Dispositif selon l'une des revendications 1 à 7, caractérisé en ce que l'électrode de chaque ensemble électrode consiste en une électrode réversible à base du couple Ag/AgCl.

9. Dispositif selon l'une des revendications 1 à 8, caractérisé en ce que le séquenceur est programmé pour actionner le relais inverseur de telle sorte que les première et deuxième voies dudit relais soient en position de travail pendant des durées Δt prédéterminées, de préférence égales, allant, par exemple, de 5 à 60 minutes et en position de repos pendant des durées δt prédéterminées, de préférence égales, allant, par exemple, de 0,5 à 30 minutes.

## Claims

1. Iontophoresis device for the transcutaneous administration of an active ingredient to a subject, ensuring accurate monitoring of the posology, of the type comprising a first electrode assembly (2) constituted by a first electrode (9) in contact with a first reservoir element (10) which is suitable, on the one hand, for containing an electrolyte incorporating the active ingredient in an at least partially ionised form or in a neutral form and, on the other hand, for ensuring, when it is placed in contact with a region (13) of the subject's skin (15), an ion-conductive continuum between the first electrode and said region, a second electrode assembly (3) constituted by a second electrode (11) in contact with a second reservoir element (12) designed to contain at least one electrolyte and to ensure, when it is placed in contact with a portion (14) of the subject's skin (15), an ion-conductive continuum between the second electrode and said portion, at least one of the first and second electrodes being a reversible electrode based on the electrochemical couple Ag/AgCl, and a generator (1) of iontophoretic electric signals connectable to each of the first and second electrodes, characterised in that it also comprises an assembly (4) for monitoring the state of advance of the transcutaneous administration of the active ingredient, which monitoring assembly comprises a programmable sequencer (5) periodically delivering control signals, a multi-path change-over relay (6) actuated by the signals delivered by the sequencer and comprising a first path and a second path simultaneously in the working position or in the resting position, the first path connecting one (9) of the electrodes of the iontophoresis device either, in the working position (16a, 17a, 19a), to a pole of the generator (1) of iontophoretic electric signals having the same sign as the electrode, or, in the resting position (16a, 18a, 19a), to a measuring output (16b), via the second path (18b, 19b, 16b), which second path, in the working position (16b, 17b, 19b), connects the measuring output to earth (26), and a measuring and/or monitoring circuit (7,33) connecting the measuring output (16b) to the other electrode (11) of the device and delivering a signal (31) representative of at least the potential difference between the electrodes (9,11) in open circuit.

2. Device according to Claim 1, characterised in that the measuring and/or monitoring circuit comprises a comparator (7) having a first input, which receives the signal (31) representative of the potential difference between the electrodes (9,11) in open circuit, and a second input, which receives a signal (33) representative of a reference potential difference, and supplying, when the said signals are equal, an output signal (34, 35 or 36), which output signal is either (i) applied (34) to the sequencer (5) in order to activate means with which the latter is equipped and which ensure that it is reset to zero and/or that the sequencer sends to the change-over relay (6) a signal (28) capable of causing the change-over relay to pass from the working position to the resting position in order to interrupt the iontophoretic circuit, or (ii) applied (35 or 36) to means with which the generator (1) of iontophoretic electric signals is equipped or which are associated with the generator and designed to ensure the stoppage of the generator or the inversion of polarity or the modification of the signals which it emits, on receiving the output signal of the comparator.

3. Device according to Claim 1 or 2, characterised in that it also comprises an amplifier (24) mounted in series with the generator of iontophoretic electric signals to produce iontophoretic electric signals having a higher peak voltage than that of the signals supplied by the generator.

4. Device according to Claim 2, characterised in that the means associated with the generator and actuated by the output signal of the comparator (7) in order to modify the signals emitted by the generator comprise an amplifier (24) mounted in series with the generator and controlled by the output signal (36) of the comparator.

5. Device according to any one of Claims 1 to 4, characterised in that it comprises a current-reversing circuit (8) associated with the generator (1) and ensuring that the polarity of the iontophoretic electric signals emitted by the generator is inverted.

6. Device according to any one of Claims 2 to 4, characterised in that the means associated with the generator and to which the output signal of the comparator (7) is applied to ensure that the polarity of the iontophoretic electric signals emitted by the generator is inverted comprise a current-reversing circuit (8) activated by the output signal (35).

7. Device according to Claim 5 or 6, characterised in that it also comprises a pulse counter designed to bring about the stoppage of the iontophoretic treatment after a predetermined number of inversions of polarity of the iontophoretic electric signals emitted by the generator.

8. Device according to any one of Claims 1 to 7, characterised in that the electrode of each electrode assembly is a reversible electrode based on the couple Ag/AgCl.

9. Device according to any one of Claims 1 to 8, characterised in that the sequencer is programmed to actuate the change-over relay in such a manner that the first and second paths of the relay are in the working position for, preferably equal, predetermined periods $\Delta t$ ranging, for example, from 5 to 60 minutes and in the resting position for, preferably equal, predetermined periods $\delta t$ ranging, for example, from 0.5 to 30 minutes.

**Patentansprüche**

1. Iontophoresevorrichtung zum transdermalen Verabreichen eines Wirkstoffs an ein Lebewesen, bei der die Dosierung präzise gesteuert wird, des Typs mit einer ersten Elektrodenanordnung (2) mit einer ersten Elektrode (9), die mit einem ersten Reservoirelement (10) in Verbindung steht, das einerseits zum Enthalten eines den Wirkstoff in zumindest zum Teil ionisierter oder in neutraler Form enthaltenden Elektrolyten und andererseits beim Kontakt mit einer Zone (13) der Haut (15) des Lebewesens zum Schaffen eines ionischen Leiterkontinuums zwischen der ersten Elektrode und der Zone geeignet ist, einer zweiten Elektrodenanordnung (3) mit einer zweiten Elektrode (11), die mit einem zweiten Reservoirelement (12) in Kontakt steht, das zum Enthalten mindestens eines Elektrolyten geeignet ist und beim Kontakt mit einem Teil (14) der Haut (15) des Lebewesens zwischen der zweiten Elektrode und dem Teil ein Leiterkontinuum herstellt, wobei mindestens eine der beiden Elektroden eine reversible Elektrode aufgrund der elektrochemischen Ag/AgCl-Kopplung ist, und mit einem Generator (1) elektrischer Iontophoresesignale, die an jede der beiden Elektroden geleitet werden können, dadurch gekennzeichnet, daß sie unter anderem die folgenden Elemente aufweist: eine Anordnung (4) zum Überwachen des Fortschritts des transdermalen Verabreichens des Wirkstoffs, wobei die Überwachungsanordnung einen programmierbaren, periodisch Befehlssignale aussendenden Sequencer (5) und einen vielpoligen Umschalter (6), der durch die vom Sequencer gelieferten Signale betätigt wird und einen ersten und einen zweiten Pfad hat, die gleichzeitig in Arbeits- oder in Ruheposition sind, aufweist, wobei der erste Pfad eine (9) der Elektroden der Iontophoresevorrichtung entweder in der Arbeitsposition (16a, 17a, 19a) mit einem Pol des Generators (1) elektrischer Iontophoresesignale mit dem gleichen Vorzeichen wie die Elektrode oder in der Ruheposition (16a, 18a, 19a) mit einem Meßausgang (16b) über den zweiten Pfad (18b, 19b, 16b) verbindet, wobei der zweite Pfad in der Arbeitsposition (16b, 17b, 19b) den Meßausgang mit Masse (26) verbindet, und eine Meß- und/oder Steuerschaltung (7,33), die den Meßausgang (16b) mit der anderen Elektrode (11) der Vorrichtung verbindet und ein Signal (31) aussendet, das mindestens der Potentialdifferenz zwischen den Elektroden (9,11) bei offener Schaltung entspricht.

2. Vorrichtung nach Anspruch 1, dadurch gekennzeichnet, daß die Meß- und/oder Steuerschaltung einen Komparator (7) aufweist, der einen ersten Eingang hat, der das mindestens der Potentialdifferenz zwischen den Elektroden (9,11) bei offener Schaltung entsprechende Signal (31) empfängt, und einen zweiten Eingang, der ein Signal (33) empfängt, das einer Referenzpotentialdifferenz entspricht, und bei Gleichheit ein Ausgangssignal (34, 35 oder 36) liefert, wobei das Ausgangssignal entweder (i) an den Sequencer (5) angelegt (34) wird, wodurch die in diesem vorhandenen Einrichtungen aktiviert werden und er zurückgestellt und/oder über den Sequencer ein Signal (28) zum Umschalter (6) geschickt wird, das den Umschalter von der Arbeitsposition in die Ruheposition bringt, wodurch die Iontophoreseschaltung unterbrochen wird, oder (ii) an die im Generator (1) elektrischer Iontophoresesignale vorhandenen oder mit ihm in Verbindung stehenden und zum Sperren des Generators oder zum Umpolen oder Modifizieren der von ihm beim Empfang des Ausgangssignals des Komparators ausgesendeten Signale geeigneten Einrichtungen angelegt (35 oder 36) wird.

3. Vorrichtung nach Anspruch 1 oder 2, dadurch gekennzeichnet, daß sie unter anderem einen Verstärker (24) aufweist, der mit dem Generator elektrischer Iontophoresesignale in Reihe geschaltet ist, zum Erzeugen elektrischer Iontophoresesignale mit einer Spitzenspannung, die größer ist als die der vom Generator erzeugten Signale.

4. Vorrichtung nach Anspruch 2, dadurch gekennzeichnet, daß die mit dem Generator in Verbindung stehenden und durch das Ausgangssignal des Komparators (7) zum Modifizieren der durch den Generator ausgesendeten Signale betätigten Einrichtungen einen Verstärker (24) aufweisen, der mit dem Generator in Reihe geschaltet ist und durch das Ausgangssignal (36) des Komparators gesteuert wird.

5. Vorrichtung nach einem der Ansprüche 1 bis 4, dadurch gekennzeichnet, daß sie eine mit dem Generator in Verbindung stehende Stromumkehrschaltung (8) aufweist, die die vom Generator ausgesendeten elektrischen Iontophoresesignale umpolt.

6. Vorrichtung nach einem der Ansprüche 2 bis 4, dadurch gekennzeichnet, daß die Einrichtungen, die mit dem Generator in Verbindung stehen und an die das Ausgangssignal des Komparators (7) zum Umpolen der vom Generator ausgesendeten elektrischen Iontophoresesignale angelegt wird, eine Stromumkehrschaltung (8) aufweisen, die durch das Ausgangssignal (35) aktiviert wird.

7. Vorrichtung nach Anspruch 5 oder 6, dadurch gekennzeichnet, daß sie außerdem einen Impulszähler aufweist, der ein Beenden der iontophoretischen Behandlung auslöst, wenn die durch den Generator ausgesendeten elektrischen Iontophoresesignale eine vorbestimmt Anzahl von Malen umgepolt wurden.

8. Vorrichtung nach einem der Ansprüche 1 bis 7, dadurch gekennzeichnet, daß die Elektrode einer jeden Elektrodenanordnung aus einer aufgrund der Ag/AgCl-Kopplung umkehrbaren Elektrode besteht.

9. Vorrichtung nach einem der Ansprüche 1 bis 8, dadurch gekennzeichnet, daß der Sequencer derart programmiert ist, daß er den Umschalter so betätigt, daß der erste Pfad und der zweite Pfad des Schalters während vorbestimmter Zeiträume $\Delta t$ in Arbeitsposition sind, die zum Beispiel zwischen 5 und 60 Minuten liegen und vorzugsweise gleich sind, und während vorbestimmter Zeiträume $\delta t$ in Ruheposition sind, die zum Beispiel zwischen 0,5 und 30 Minuten liegen und vorzugsweise gleich sind.